# EUROPEAN PATENT SPECIFICATION

(11) **EP 2 991 581 B1**
(45) Date of publication and mention of the grant of the patent: **06.06.2018**
(21) Application number: 14791418.8
(22) Date of filing: 14.04.2014
(51) Int. Cl.: A61F 2/00, A61F 2/95, A61M 25/10, A61F 2/958

(54) **STENT DELIVERY APPARATUS**
STENTVORRICHTUNG
APPAREIL PERMETTANT LA POSE ET LE DÉPLOIEMENT D'UNE ENDOPROTHÈSE

(30) Priority: 03.05.2013 US 201361819337 P
(43) Date of publication of application: 09.03.2016
(73) Proprietor: Amaranth Medical PTE., Singapore Science Park II, Singapore 117610 (SG)
(72) Inventor: RAMZIPOOR, Kamal, Fremont, California 94539 (US); LEE, Chang Y., San Jose, California 95112 (US); ESTRADA, Edward A., Menlo Park, California 94025 (US)
(74) Representative: J A Kemp
(86) International application number: PCT/US2014/034038
(87) International publication number: WO 2014/179022

(56) References cited:
- WO-A1-2004/047685
- WO-A2-00/57945
- US-A- 5 639 274
- US-A- 6 106 530
- US-A1- 2010 274 343
- US-A1- 2010 274 344
- US-A1- 2011 099 785
- US-B1- 6 395 008
- US-B1- 6 585 747
- US-B2- 8 057 526
- US-B2- 8 152 819

## Description

### FIELD OF THE INVENTION

The present invention relates generally to stent or scaffold delivery apparatus. More particularly, the present invention relates to apparatus for providing a smooth transition between a crimped stent and inflation balloon for facilitating intravascular delivery and deployment of the stent or scaffold.

### BACKGROUND OF THE INVENTION

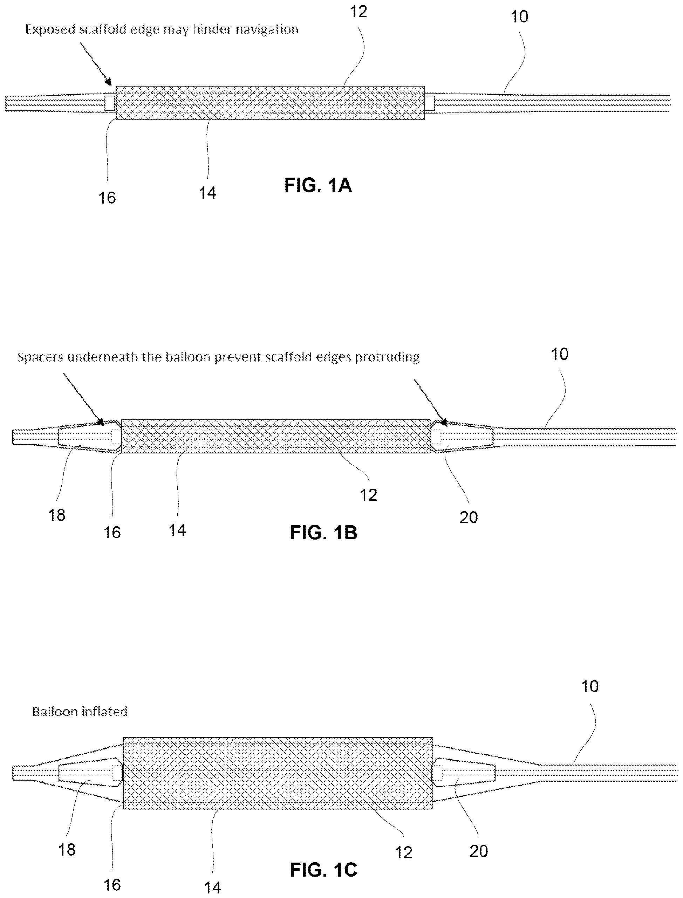

When a scaffold or stent **12** is crimped upon an inflation balloon **14** for delivery via a catheter **10** within the vasculature, the edges **16** of the scaffold **12** may protrude radially at the balloon-scaffold transition, as shown in the side view of Fig. 1A, and prevent smooth intravascular navigation to the target site.

Accordingly, it is desirable to provide for a smooth transition region between the balloon or catheter and the scaffold or stent crimped or otherwise secured upon the balloon.

US 2011/099785 A1 discloses a stent delivery system. The system has a balloon disposed about at least a portion of a catheter, the balloon having a first end and a second end and a working length therebetween, the first end and the second end each including a tapered portion, each tapered portion being attached to the catheter. The balloon is inflatable from a collapsed configuration to an inflated configuration. The stent has a first end and a second end, the first end and the second end each including a tapered portion. The stent is disposed over the balloon such that at least a portion of the tapered first end and the tapered second end of the balloon are covered by the stent.

### SUMMARY OF THE INVENTION

According to the present invention there is provided the scaffold delivery apparatus of claim 1.

Additional aspects of the invention are set out in the dependent claims.

### BRIEF DESCRIPTION OF THE DRAWINGS

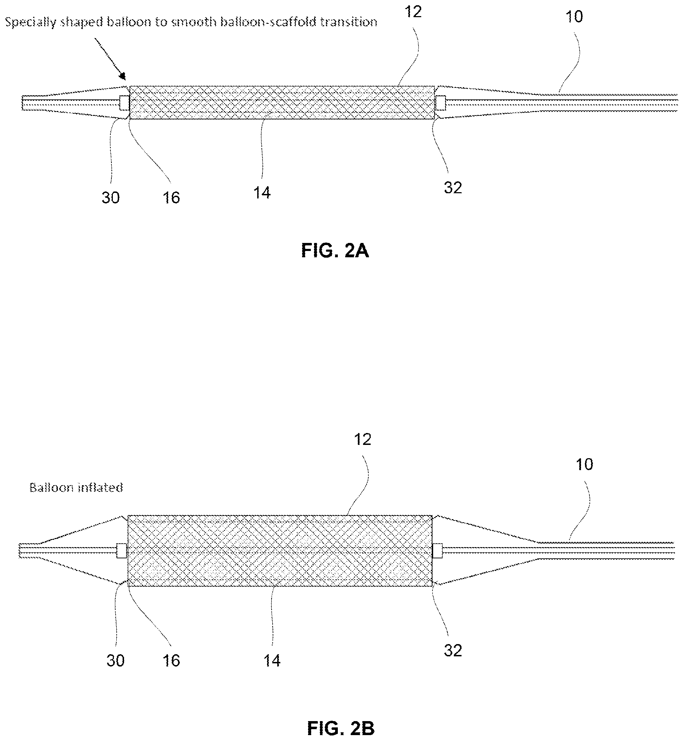

The present invention will now be described, by way of example only, with reference to the accompanying drawings. Although the variations of Figs. 1B & 1C and Figs. 2A & 2B illustrates scaffolds which do not define a distal tapered edge and a proximal tapered edge, these variations nevertheless represent technical background that is useful for understanding the scaffold delivery apparatus of the present invention.
Fig. 1A shows a side view of a scaffold crimped upon a balloon illustrating the exposed scaffold edges which may hinder navigation of the assembly through the vasculature.
Figs. 1B and 1C show side views of one variation of an assembly having tapered spacers underneath or within the distal and proximal portions of the inflation balloon which prevent radial exposure of the scaffold edges.
Figs. 2A and 2B show side views of another variation of an assembly where the distal and proximal portions of the inflation balloon abutting the scaffold may be specially shaped in a tapered manner to prevent radial exposure of the scaffold edges.
Figs. 3A and 3B show side views of yet another variation of an assembly where the distal and proximal portions of the scaffold may be shaped or tapered to present a smooth transition between the catheter, inflation balloon, and scaffold.

### DETAILED DESCRIPTION OF THE INVENTION

For scaffolds **12** with wall thicknesses greater than 50 µm, it is possible to improve intravascular navigation by creating a smoother transition from balloon-to-scaffold. One method of creating this smooth transition is to place spacers underneath or within the balloon **14** that will increase the diameter of balloon **14** just prior and/or just after the scaffold, as shown in the side view of Fig. 1B. A distal spacer **18** which is tapered to widen radially from its distal end to its proximal end may be positioned distal to the scaffold **12.** A similar proximal spacer **20** which is tapered to narrow radially from its distal end to its proximal end may be positioned proximal to the scaffold **12** as well. The widened ends of the spacers **18**, **20** may have a diameter which is similar or the same as the diameter of the crimped scaffold **12** secured upon the balloon **14** to prevent the scaffold edges **16** from protruding beyond the spacers **18**, **20** during intravascular delivery. Alternatively, depending on the crimped diameter of the scaffold **12** the diameter of the crimped balloon **14** at the spacer regions can range from one wall thickness less than the crimped diameter of the scaffold **12** to two wall thickness greater than crimped diameter of the scaffold **12**.

When the scaffold **12** is to be deployed, the inflation balloon **14** may be simply expanded as normally done to expand the scaffold **12** beyond the diameter of the spacers **18**, **20**, as shown in the side view of Fig. 1C.

Another variation is illustrated in the side views of Figs. 2A and 2B which show an inflation balloon **14** which is specially configured to define a step **30**, **32** in the balloon **14** to accommodate the diameter change due to scaffold wall thickness. The distal step **30** may be defined by the balloon **14** distal to the scaffold **12** and the proximal step **32** may be defined by the balloon **14** proximal to the scaffold **12** such that the steps **30**, **32** abut the scaffold **12** in a tapered manner to present a smooth balloon-scaffold transition. The balloon **14** may be inflated normally for deployment into the body lumen.

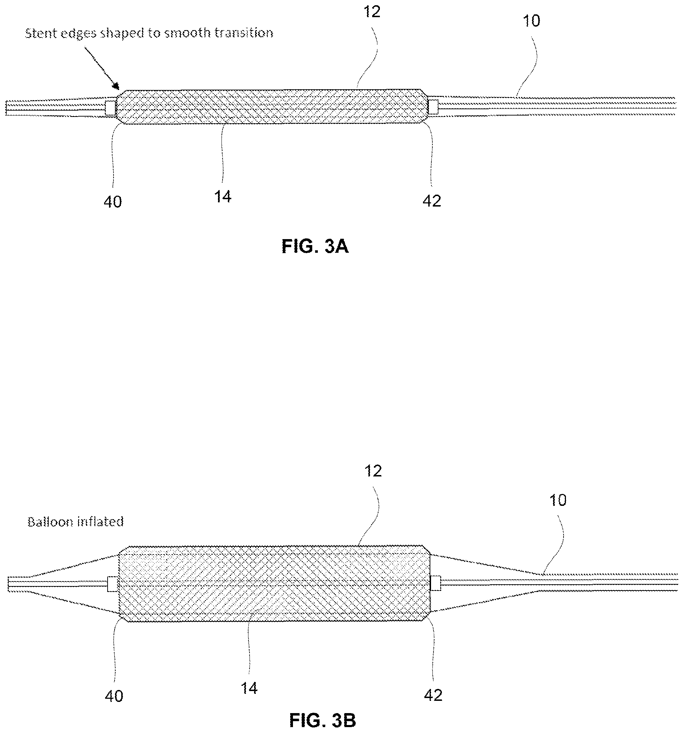

Figs. 3A and 3B show side views of yet another variation where the distal **40** and proximal edges **42** of the scaffold **12** are tapered. The transitioning scaffold edges **40**, **42** can be created using methods including, e.g., angled laser beam, profile machining, control depth profile machining, using laser beam or other mechanisms, injection molding process, abrasive or material removal process, as well as other material removal/deposit processes. The balloon **14** may be simply inflated as normal for stent deployment, as shown in Fig. 3B.

Lastly, the stent **12** can also be made thinner to smooth the balloon-scaffold transition. The thinned stent **12** as well as each of the transition features described herein may be used in any number of combinations. For example, the spacers **18, 20** may be used in combination with the tapered balloon edges **30**, **32** in a single assembly. Alternatively, these features may also be used in combination with the tapered scaffold edges **40**, **42** in any variation.

Moreover, the transition features described herein may be used with any number of scaffolds or stent structures and particularly with polymeric substrate and stent assemblies as described in U.S. Pat. Apps. 10/867,617 filed June 15, 2004 (U.S. Pub. 2005/0021131); 13/476,853 filed May 21, 2012 (U.S. Pub. 2012/0232643); 13/476,858 filed May 21, 2012 (U.S. Pub. 2012/0232644); 12/541,095 filed August 13, 2009 (U.S. Pub. 2010/0042202); U.S. Pats. 8,206,635; 8,206,636; and 8,309,023.

Modification of the above-described methods and devices for carrying out the invention, and variations of aspects of the invention that are obvious to those of skill in the arts, are intended to be within the scope of the claims. Moreover, various combinations of aspects between examples are also contemplated and are considered to be within the scope of the claims as well.

## Claims

1. A scaffold delivery apparatus, comprising:
a catheter (10) having an inflatable balloon (14) thereupon, wherein the inflatable balloon comprises a distal portion, a proximal portion, and a central portion in between the distal portion and the proximal portion;
an expandable scaffold (12) secured only upon the central portion of the inflatable balloon, wherein the central portion is substantially radially constant throughout the entire length of the central portion;
wherein the distal portion of the inflatable balloon defines a radially tapered transition adjacent to a distal end of the scaffold and the proximal portion of the inflatable balloon defines a radially tapered transition adjacent to the proximal end of the scaffold, and
wherein the scaffold defines a distal tapered edge (40) and a proximal tapered edge (42).

2. The apparatus of claim 1 wherein the catheter comprises an intravascular catheter.

3. The apparatus of claim 1 wherein the expandable scaffold has a wall thickness greater than 50 µm.

4. The apparatus of claim 1 wherein the expandable scaffold is configured to be crimped upon the inflatable balloon when the inflatable balloon is in a deflated state.

5. The apparatus of claim 1 wherein the distal portion and the proximal portion of the inflatable balloon each define a step in the inflatable balloon, which step is sized to accommodate the diameter difference between the inner and outer walls of the scaffold due to the wall thickness of the scaffold.

6. The apparatus of claim 5 wherein the steps abut the scaffold in a tapered manner to present a smooth balloon-scaffold transition.

## Patentansprüche

1. Gerüst-Abgabevorrichtung, umfassend:
einen Katheter (10) mit einem aufblasbaren Ballon (14) darauf, wobei der aufblasbare Ballon einen distalen Abschnitt, einen proximalen Abschnitt und einen zentralen Abschnitt zwischen dem distalen Abschnitt und dem proximalen Abschnitt umfasst;
ein ausdehnbares Gerüst (12), das nur auf dem zentralen Abschnitt des aufblasbaren Ballons befestigt ist, wobei der zentrale Abschnitt im Wesentlichen radial konstant durch die gesamte Länge des zentralen Abschnitts ist;
wobei der distale Abschnitt des aufblasbaren Ballons einen sich radial verjüngenden Übergang aufweist, der an ein distales Ende des Gerüsts angrenzt, und der proximale Abschnitt des aufblasbaren Ballons einen sich radial verjüngenden Übergang aufweist, der an das proximale Ende des Gerüsts angrenzt, und
wobei das Gerüst eine distale, sich verjüngende Kante (40) und eine proximale, sich verjüngende Kante (42) definiert.

2. Vorrichtung nach Anspruch 1, wobei der Katheter einen intravaskulären Katheter umfasst.

3. Vorrichtung nach Anspruch 1, wobei das ausdehnbare Gerüst eine Wandstärke größer als 50 µm aufweist.

4. Vorrichtung nach Anspruch 1, wobei das ausdehnbare Gerüst konfiguriert ist, um auf den aufblasbaren Ballon geheftet zu werden, wenn sich der aufblasbare Ballon in einem entleerten Zustand befindet.

5. Vorrichtung nach Anspruch 1, wobei der distale Abschnitt und der proximale Abschnitt des aufblasbaren Ballons jeweils eine Stufe in dem aufblasbaren Ballon definieren, welche Stufe bemessen ist, um den Durchmesserunterschied zwischen den inneren und äußeren Wänden des Gerüsts aufgrund der Wandstärke des Gerüsts zu berücksichtigen.

6. Vorrichtung nach Anspruch 5, wobei die Stufen auf eine sich verjüngende Weise an das Gerüst anschließen, um einen glatten Ballon-Gerüst-Übergang darzustellen.

## Revendications

1. Appareil de pose de support comprenant :
un cathéter (10) sur lequel se trouve un ballonnet gonflable (14), le ballonnet gonflable comprenant une partie distale, une partie proximale et une partie centrale entre la partie distale et la partie proximale ;
un support déployable (12) arrimé uniquement sur la partie centrale du ballonnet gonflable, la partie centrale étant sensiblement radialement constante sur toute la longueur de la partie centrale ;
la partie distale du ballonnet gonflable définissant une transition radialement effilée adjacente à une extrémité distale du support et la partie proximale du ballonnet gonflable définissant une transition radialement effilée adjacente à l'extrémité proximale du support, et
le support définissant un bord effilé distal (40) et un bord effilé proximal (42).

2. Appareil de la revendication 1, dans lequel le cathéter comprend un cathéter intravasculaire.

3. Appareil de la revendication 1, dans lequel le support déployable présente une épaisseur de paroi supérieure à 50 µm.

4. Appareil de la revendication 1, dans lequel le support déployable est conçu pour être fixé par sertissage sur le ballonnet gonflable lorsque le ballonnet gonflable se trouve dans un état dégonflé.

5. Appareil de la revendication 1, dans lequel la partie distale et la partie proximale du ballonnet gonflable définissent chacune un pallier dans le ballonnet gonflable, le pallier étant dimensionné pour adapter la différence de diamètre entre les parois interne et externe du support en raison de l'épaisseur de paroi du support.

6. Appareil de la revendication 5, dans lequel le pallier bute contre le support de manière effilée pour présenter une transition ballonnet-support lisse.
